(19) Europäisches Patentamt
European Patent Office
Office européen des brevets

(11) **EP 2 467 027 B1**

(12) **EUROPEAN PATENT SPECIFICATION**

(45) Date of publication and mention
of the grant of the patent:
**01.03.2017 Bulletin 2017/09**

(51) Int Cl.:
*A23G 3/34* (2006.01)     *A61K 9/00* (2006.01)
*A61K 9/16* (2006.01)     *A23K 40/10* (2016.01)
*A23K 40/20* (2016.01)     *A23K 20/163* (2016.01)
*A23P 10/28* (2016.01)     *A23L 29/30* (2016.01)
*A23L 27/30* (2016.01)     *A23G 3/42* (2006.01)
*A61K 9/20* (2006.01)

(21) Application number: **10736985.2**

(22) Date of filing: **12.07.2010**

(86) International application number:
**PCT/EP2010/004224**

(87) International publication number:
**WO 2011/020527 (24.02.2011 Gazette 2011/08)**

(54) **PROCESS FOR COMPRESSING ISOMALT**

Verfahren zur Verdichtung von Isomalt

Procédé de compression d'isomalt

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**

(30) Priority: **18.08.2009 EP 09010596**

(43) Date of publication of application:
**27.06.2012 Bulletin 2012/26**

(73) Proprietor: **Cargill, Incorporated
Wayzata, MN 55391 (US)**

(72) Inventors:
• **BOGHMANS, Catherine, Patricia L.
B-1852 Beigem (BE)**
• **MEEUS, Liesbeth, Maria, Fernande
B-3078 Everberg (BE)**

(74) Representative: **Elseviers, Myriam et al
Cargill R&D Centre Europe BVBA
Bedrijvenlaan 9
2800 Mechelen (BE)**

(56) References cited:
EP-A1- 2 095 815          WO-A1-2005/115342
WO-A1-2009/020268          WO-A2-2009/015791
US-B1- 6 849 286

• BOLHUIS G K ET AL: "Compaction properties of isomalt" EUROPEAN JOURNAL OF PHARMACEUTICS AND BIOPHARMACEUTICS, ELSEVIER SCIENCE PUBLISHERS B.V., AMSTERDAM, NL, vol. 72, no. 3, 1 August 2009 (2009-08-01), pages 621-625, XP026218302 ISSN: 0939-6411 [retrieved on 2009-03-25]
• NDINDAYINO F ET AL: "Characterization and evaluation of isomalt performance in direct compression" INTERNATIONAL JOURNAL OF PHARMACEUTICS, ELSEVIER BV, NL, vol. 189, no. 1, 28 October 1999 (1999-10-28), pages 113-124, XP002486902 ISSN: 0378-5173

**Description**

Technical field

The present invention relates to the preparation of an isomalt containing composition suitable for tabletting.

Background of the invention

With the present interest in the use of sugar-free and/or low calorie products, tablets for pharmaceutical, confectionery or food applications are mostly made with sugar alcohols, such as xylitol, maltitol, sorbitol, mannitol and erythritol.

The tablet does not only contain the drug or a reagent, it also contains other ingredients which act as fillers, such as lactose or phosphates; lubricating agents, such as talc, stearic acid or paraffin and disintegrating agents, such as carboxymethyl-cellulose or starch. For confectionery purposes the tablets often include aroma's and colorants at low concentration.

US 6,224,904 relates to a compressed formulation containing 1-glucopyranosyl-sorbitol or a mixture of sweetening agents composed of 1-glucopyranosyl-sorbitol, 6-glucopyranosyl-sorbitol and 1-glucopyranosyl-mannitol. A mixture with increased 1-glucopyranosyl-mannitol is used for the production of chewable tablets.

US 6,849,286 relates to a method of producing an improved compressed product wherein the agglomeration of the ingredients is induced. The invention provides that according to a first procedure step the educt, namely isomaltulose, isomalt and/or the mixtures containing 6-glucopyranosyl-sorbitol and 1-glucopyranosyl-mannitol, is milled while dry and the particles should have a maximum size of 100 $\mu$m.

Compaction properties of isomalt by G., K., Bolhuis et. al relates to the compaction properties of different types of isomalt.

There is a further interest for having an improved method for preparing a compressed product containing isomalt and which can be used as an excipient in tablets.

Summary of the invention

The current invention relates to a method of producing a compressed product of a dry isomalt wherein 6-glucopyranosyl-sorbitol is present in a weight percentage from 43% to 57% and 1-glucopyranosyl-mannitol in a weight percentage from 57% to 43%, and said method is comprising the following steps:

    a. Agglomerating the isomalt having a volume mean dimater not smaller than 250 $\mu$m with a liquid binder,
    b. At the same time or thereafter drying of the agglomerate,
    c. Reducing volume mean diameter of agglomerate to a volume mean diameter smaller than 180 $\mu$m, preferably smaller than 150 $\mu$m, more preferably smaller than 110 $\mu$m,
    d. Compressing the agglomerate with reduced volume mean diameter into a compressed product.

In the current invention the liquid binder is water, liquid sorbitol, maltodextrin and water, and/or mixtures thereof, and the agglomeration is applying a fluidized bed.

Detailed description of the invention

The current invention relates to a method of producing a compressed product of a dry isomalt wherein 6-glucopyranosyl-sorbitol is present in a weight percentage from 43% to 57% and 1-glucopyranosyl-mannitol in a weight percentage from 57% to 43%, and said method is comprising the following steps:

    e. Agglomerating the isomalt having a volume mean dimater not smaller than 250 $\mu$m with a liquid binder,
    f. At the same time or thereafter drying of the agglomerate,
    g. Reducing volume mean diameter of agglomerate to a volume mean diameter smaller than 180 $\mu$m, preferably smaller than 150 $\mu$m, more preferably smaller than 110 $\mu$m,
    h. Compressing the agglomerate with reduced volume mean diameter into a compressed product.

Isomalt is understood to refer to an almost equimolar mixture of 6-glucopyranosyl-sorbitol (6-GPS) and 1-glucopyranosyl-mannitol (1-GPM), and the weight percentage can vary between 43 to 57% of 6-GPS to 57% to 43% of 1-GPM. The isomalt may further comprise minor amounts of other substances such mannitol, sorbitol, hydrogenated or non-hydrogenated oligosaccharides as well as optionally glucose, fructose and/or sucrose, trehalulose, isomaltulose or isomaltose.

The isomalt used for the agglomeration step has a volume mean diameter not smaller than 250 $\mu$m preferably not smaller than 300 $\mu$m, more preferably not smaller than 400 $\mu$m.

Preferably, the volume mean diameter is not bigger than 1000 $\mu$m. Even when starting from isomalt with an even smaller volume mean diameter, such as not smaller than 150 $\mu$m, the process still required the step of reducing the volume mean diameter of agglomerate to a volume mean diameter smaller than 110 $\mu$m, preferably smaller than 90 $\mu$m.

While adding a liquid binder, the product is agglomerated and this agglomerate can be dried during agglomeration or thereafter.

Agglomeration (granulation) methods can be divided in two basic types, namely wet methods, which use a liquid in the process, and dry methods in which no liquid is used. Wet granulation is most often used and involves many steps, including: agglomerating (granulating) of dry primary powder particles of active ingredients and excipients in the presence of a granulating fluid upon agitation using low-shear or high-shear mixers or fluidized beds, wet sieving (wet screening) to remove larger lumps, drying the granulated product, and milling or sieving (screening) the dried granulated product to achieve a granulated product having the desired granule size distribution. The obtained granulated product may subsequently be tabletted.

Surprisingly, it was found that the isomalt having a volume mean diameter of not smaller than 250 $\mu$m can directly be agglomerated into an agglomerate. No milling step is required.

In order to obtain about uniform sized particles, the volume mean diameter of the dried agglomerate is reduced to a volume smaller than 180 $\mu$m, preferably smaller than 150 $\mu$m, more preferably smaller than 110 $\mu$m, most preferably smaller than 90 $\mu$m. The volume mean diameter is measured by laser technology.

Any method is applicable for reducing the volume mean diameter of agglomerate to a volume mean diameter smaller than 180 $\mu$m. The granules (agglomerate) formed in step c) of the current process are pressed through a sieve of a predetermined size. Preferably a screening machine, more preferably an oscillating screening machine is applied for this sieving. At the same time or thereafter the product is dried.

Any drier type can be applied for drying of the granules, but preferably a fluid bed is applied for this purpose. These uniform sized particles are compressed to obtain a compressed product in a typical tabletting equipment.

The current invention relates to a method wherein the liquid binder is water, liquid sorbitol, maltodextrin and water, and/or mixtures thereof.

The liquid binder is added in an amount from 2 to 25% based upon the dry matter of isomalt.

Whenever the liquid binder is water, it is added in an amount of 2 to 10%, preferably 4 to 8% based upon the dry matter of the isomalt. The liquid binder may also be liquid sorbitol applied in a dry matter concentration of at least 50%, preferably 60%, more preferably at least 70%.

The liquid binder (= total amount of liquid sorbitol) is added in an amount of 2% to 10%, preferably 4 to 7% based upon the dry matter of the isomalt.

Maltodextrin can be added in dry form and water is added during granulation. Alternatively, maltodextrin and water is used as a liquid binder. Maltodextrin is a polysaccharide that is used as a food additive. It is hydrolysate produced from starch and consists of D-glucose units connected in chains of variable length. The glucose units are linked with $\alpha(1{\rightarrow}4)$ bonds.

Maltodextrin is typically composed of mixtures of chains that vary from three to nineteen glucose units long. Maltodextrins are classified by DE (dextrose equivalent) and have a DE of 20 or lower, mostly between 5 to 20. The total amount of liquid binder (= maltodextrin and water) is from 15 to 25%, preferably from 17% to 23% based upon the dry matter of the isomalt. The liquid binder which is consisting of maltodextrin and water, is containing maltodextrin in an amount of 15% to 20%, preferably about 18% based upon total weight of liquid binder.

In a preferred embodiment, the method according to the current invention is applying a fluidized bed for the agglomeration. The drying can be performed in the fluidized bed as well, when the bed is heated to a temperature above room temperature.

Additives other than isomalt or auxiliary substances such as lubricants are added between the drying and the compressing step and before/after the step of reducing the volume mean diameter. Additives other than isomalt can be added to preserve flavor or enhance taste and appearance. Such substances may include sweeteners, flavorings, taste substances and coloring agents, food-compatible acids, disintegrants, monosaccharides, disaccharides, monosaccharide alcohols, disaccharide alcohols different from isomalt, starch, starch derivatives, pectins, polyvinylpyrrolidone, cellulose, cellulose derivatives, intense sweeteners, stearic acid or the salts thereof, or inulin. Preferably, as a lubricant agent in tablet formation, magnesium stearate, calcium stearate, stearic acid, sucrose fatty acid esters, and/or talc and the like can be added according to needs. Furthermore surface active agents such as sodium lauryl sulfate, propylene glycol, sodium dodecanesulfonate, sodium oleate sulfonate, and sodium laurate mixed with stearates and talc, sodium stearyl fumarate, sucrose fatty acid esters, and the like can be added according to needs. These additives other than isomalt or auxiliary substances may be occasionally added during the agglomeration step as well.

If compressed products (tablets) are prepared for pharmaceutical applications an active ingredient different from isomalt and fillers, lubricating agents or disintegrating agents are added if needed. Such an active ingredient is different form isomalt and may be a substance in a pharmaceutical drug or cosmetics, detergents, fertilizer or agrochemical products that is biologically active. Preferably the active ingredient is added during the agglomeration.

The term "tablet", as used herein, includes any tablet, in particular tablets in any form, shape and of any physical, chemical or sensory property, and tablets for any route of administration, indication and application. The tablet produced according to the invention is a chewable tablet. A chewable tablet according to the present invention is a soft tablet where chewing helps to break the tablet particles and release the active ingredient, flavor, aroma or the like, in the mouth before swallowing. Chewable tablets are designed to be mechanically disintegrated in the mouth. A chewable tablet dosage form can be a soft pill, tablet, gum and more recently "chewy squares". The tablet hardness is a highly important property of a chewable tablet comprising active ingredient(s) and having desirable chewability properties.

The compressed product (tablet) obtained according to the method of the current invention, is showing a hardness of from 120 to 300 N at a compression force of from 10 to 20 kN, preferably a hardness of from 125 to 280 N, more preferably from 130 to 270 N for tablets having a surface of 1 cm$^2$, a diameter of 11.3 mm and a weight of 350 mg.

Said tablets can be applied in food applications, feed, pharma applications, cosmetics, detergents, fertilizer or agrochemical products. In fact, without being limiting, the compressed product of the current invention can be used in food products, animal feed, health food, dietetic products, animal medicine, with bath agent, in agrochemical products, with fertilizer, with plant granules, with plant seeds or seed grains, and any other product being it ingested by humans and/or animals or any other product which can benefit from the improved properties of the compressed product of the current invention. The compressed product of the current invention can be used as carrier for additives based on enzymes or microorganisms, detergent tablets, vitamins, flavors, perfumes, acids, sweeteners, different from isomalt or various active ingredients with medicinal or non-medicinal applications. Eventually mixtures of additives different from isomalt can be applied.

If tablets are prepared for food (confectionery) applications than in general up to about 99 % (w/w) consists of the isomalt,and aroma, colorants, flavors and a lubricating agent, are added.

The invention will hereunder be illustrated in the form of the following examples.

Examples

Methods for evaluating granule and tablet properties

The granules were characterized by their volume mean diameter (size distribution).

The following measurement method was employed.

Size distribution. Size distribution was determined according to the European Pharmacopoeia VI Test method 2.9.31 using a laser light particle sizer, type Helos KF - Rodos T4.1, of Sympatec GmbH (Germany). The particle size was analysed by laser light diffraction.

The tablets were characterized by their hardness. For each compression force, 10 tablets for hardness were analyzed and mean values were calculated. The following measuring method was employed.

Hardness. Hardness, i.e. the diametral crushing strength, was determined according to the European Pharmacopoeia VI Test method 2.9.8 Resistance to crushing of tablets by using a conventional pharmaceutical hardness tester (hardness tester model Multicheck V, available from Erweka GmbH (Germany)). In order to compare values across different size tablets, the breaking strength was normalized for the area of the break. The normalized value, expressed as N/mm$^2$, is herein referred to as tensile strength (Ts) and calculated as follows:

$$Ts = 2H/\pi TD,$$

wherein H is the hardness, T the thickness and D the diameter of the tablet. For each compression force, 10 tablets were analyzed on hardness (H), thickness (T) and diameter (D).

Example 1

The bed of the fluid bed (Aeromatic-Fielder GEA - Strea-1) was filled with 300 g (Cargill C*PharmIsoMaltidex™ new grade 2009, having a volume mean diameter of 386 $\mu$m) and 25 g water was sprayed onto the powder at 1 Bar, at 2.8 g/min at a bed temperature of 60°C.

The drying of the granules was done on the same equipment for additionally 60 minutes at 50°C.

The dried granules were screened in the granulator (Erweka (FGS + AR400E) over a sieve of 0.125 mm to 0.250 mm for 10 to 15 minutes at 100 turns per minute. The volume mean diameter of the granules was 78 $\mu$m.

The dry sieved granules were then blended with 1 % of magnesium stearate in a Pharmatech Equipment at 28 rpm.

Example 2

The granulated product obtained in example 1 was then tabletted in a tabletting machine (Korsch - PH100) at compression forces varying from 5 kN to 30 kN.

Tablets had a surface of 1 cm$^2$, the diameter of the tablet was 11.3 mm and the weight is 350 mg.

The thus obtained tablets were analyzed as follows:

Hardness comparison

| Compression Force (kN) | Product from example 2 (N) |
|---|---|
| 10 | 130 |
| 15 | 208 |
| 20 | 274 |

Example 3

Example 1 and 2 were repeated but instead of using isomalt (Cargill C*PharmIsoMaltidex™ new grade 2009) isomalt (C*IsoMaltidex 16506 from Cargill) was applied. The recipe, the procedure as well as the outcome of the experiments was exactly the same as laid out in example 1 and 2.

**Claims**

1. A method of producing a compressed product of a dry isomalt wherein 6-glucopyranosyl-sorbitol is present in a weight percentage from 43 to 57% and 1-glucopyranosyl-mannitol in a weight percentage from 57% to 43% and said method is comprising the following steps:

   a. Agglomerating the isomalt having a volume mean diameter not smaller than 250 $\mu$m with a liquid binder,
   b. At the same time or thereafter drying of the agglomerate,
   c. Reducing volume mean diameter of agglomerate to a volume mean diameter smaller than 180 $\mu$m, preferably smaller than 150 $\mu$m, more preferably smaller than 110 $\mu$m,
   d. Compressing the agglomerate with reduced volume mean diameter into a compressed product.

2. A method according to claim 1 **characterized in that** liquid binder is water, liquid sorbitol, maltodextrin and water, and/or mixtures thereof.

3. A method according to claim 1 **characterized in that** the liquid binder is present in an amount of from 2 to 25% based upon the dry matter of isomalt.

4. A method according to anyone of claims 1 to 3 **characterized in that** the agglomeration is applying a fluidized bed.

5. A method according to anyone of claims 1 to 4 **characterized in that** active ingredient different from isomalt is added in step a).

6. A method according to anyone of claims 1 to 5 **characterized in** additives different from isomalt and/or flavors are added between step c) and d).

**Patentansprüche**

1. Verfahren zur Herstellung eines verdichteten Produkts eines trockenen Isomalts, wobei 6-Glucopyranosyl-sorbit in einem Gewichtsprozentsatz von 43 bis 57% und 1-Glucopyranosyl-mannit in einem Gewichtsprozentsatz von 57% bis 43% vorliegt und wobei das Verfahren die nachstehenden Schritte umfasst:

a. Agglomerieren des Isomalts, das einen Volumen-mittleren Durchmesser von nicht geringer als 250 $\mu$m aufweist, mit einem flüssigen Bindemittel,
b. Gleichzeitig oder danach Trocknen des Agglomerats,
c. Vermindern des Volumen-mittleren Durchmessers vom Agglomerat auf einen Volumen-mittleren Durchmesser geringer als 180 $\mu$m, vorzugsweise geringer als 150 $\mu$m, bevorzugter geringer als 110 $\mu$m,
d. Verdichten des Agglomerats mit vermindertem Volumen-mittlerem Durchmesser zu einem verdichteten Produkt.

**2.** Verfahren nach Anspruch 1, **dadurch gekennzeichnet, dass** flüssiges Bindemittel Wasser, flüssiges Sorbit, Maltodextrin und Wasser und/oder Gemische davon ist.

**3.** Verfahren nach Anspruch 1, **dadurch gekennzeichnet, dass** das flüssige Bindemittel in einer Menge von 2 bis 25%, basierend auf der Trockenmasse von Isomalt, vorliegt.

**4.** Verfahren nach einem der Ansprüche 1 bis 3, **dadurch gekennzeichnet, dass** die Agglomeration eine Wirbelschicht bzw. ein Fluidatbett verwendet.

**5.** Verfahren nach einem der Ansprüche 1 bis 4, **dadurch gekennzeichnet, dass** ein von Isomalt verschiedener Wirkstoff in Schritt a) zugegeben wird.

**6.** Verfahren nach einem der Ansprüche 1 bis 5, gekennzeichnet zwischen Schritt c) und d) in der Zugabe von von Isomalt verschiedenen Additiven und/oder Aromen.


**Revendications**

**1.** Procédé pour produire un comprimé sec d'isomalt contenant du sorbitol 6-glucopyranosyle selon un pourcentage en poids compris entre 43% et 57% et du mannitol 1-glucopyranosyle selon un pourcentage en poids compris entre 57% et 43%, ledit procédé comprenant les étapes suivantes :

a. agglomération de l'isomalt dont le diamètre volumique n'est pas inférieur à 250 $\mu$m avec un liant liquide,
b. séchage simultané ou ultérieur de l'aggloméré,
c. réduction du diamètre volumique moyen à un diamètre volumique moyen inférieur à 180 $\mu$m, de préférence inférieur à 150 $\mu$m, plus préférentiellement inférieur à 110 $\mu$m,
d. compression de l'aggloméré présentant un diamètre volumique moyen réduit pour former un produit comprimé.

**2.** Procédé selon la revendication 1 **caractérisé en ce que** le liant liquide est de l'eau, du sorbitol liquide, de la maltodextrine, et/ou leurs mélanges.

**3.** Procédé selon la revendication 1 **caractérisé en ce que** le liant liquide est présent en une quantité allant de 2% à 25% par rapport à la matière sèche d'isomalt.

**4.** Procédé selon l'une quelconque des revendications 1 à 3 **caractérisé en ce que** l'agglomération utilise un lit fluidisé.

**5.** Procédé selon l'une quelconque des revendications 1 à 4 **caractérisé en ce qu'**un ingrédient actif différent de l'isomalt est ajouté à l'étape a).

**6.** Procédé selon l'une quelconque des revendications 1 à 5 **caractérisé en ce que** des ingrédients différents de l'isomalt et/ou des agents de saveur sont ajoutés entre les étapes c) et d).

**EP 2 467 027 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6224904 B

- US 6849286 B